# EUROPEAN PATENT APPLICATION

(11) **EP 4 417 199 A1**
(43) Date of publication of application: **21.08.2024**
(21) Application number: 22881016.4
(22) Date of filing: 11.10.2022
(51) Int. Cl.: A61K 31/202, A23D 9/00, A23L 33/115, A61K 31/232, A61P 11/00, A61P 11/02, A61P 17/04, A61P 27/02, A61P 37/08

(54) **ANTI-ALLERGIC AGENT**

(30) Priority: 13.10.2021 JP 2021168412
(71) Applicant: Kao Corporation, Tokyo 103-8210 (JP)
(72) Inventor: SAYAMA, Keimon, Tokyo 113-8421 (JP); FUKAGAWA, Satoko, Tokyo 113-8421 (JP); ISHIKAWA, Junko, Tokyo 113-8421 (JP); SASAKI, Aya, Tokyo 131-8501 (JP)
(74) Representative: Vossius & Partner Patentanwälte Rechtsanwälte mbB
(86) International application number: PCT/JP2022/037896
(87) International publication number: WO 2023/063321

(57) **Abstract**

Provided is an anti-allergic agent. The anti-allergic agent comprises, as an active ingredient, a fat or oil in which a content of α-linolenic acid in fatty acids constituting the fat or oil is 40 mass% or more, and a content of diacylglycerol is 25 mass% or more.

## Description

### Field of the Invention

The present invention relates to an anti-allergic agent.

### Background of the Invention

The human body is equipped with a mechanism called immunity to protect itself from infectious microorganisms, foreign substances, and the like. An allergy is where this immune system becomes abnormal, causing symptoms such as sneezing, rashes, and breathing difficulties.

In general, the onset mechanism of allergy is classified into four types of reactions, from type I to type IV. Of these, type I is the one causing the main allergies such as hay fever, allergic rhinitis, bronchial asthma, allergic conjunctivitis, food allergies, and anaphylactic shock.

In a type I reaction, helper T cells are activated by the presented antigen (allergen), which increases the production of cytokines such as IL-4 (interleukin 4). In addition, IgE antibodies produced by B cells bind to high-affinity IgE receptors (FcεR1) on mast cells (mastocytes) present in tissues and on basophils in the blood. When the antigen re-enters the body in such a sensitized state, it causes an antigen-antibody reaction with the IgE antibodies bound on mast cells and basophils. This stimulus produces a series of reactions within the cells, which releases chemical messengers such as histamine outside the cells, causing smooth muscle contraction, increased capillary permeability, increased glandular secretion, and the like, and results in the development of various allergic symptoms.

Examples of the factors contributing to the onset or aggravation of allergies include individual factors including genetic predisposition, and environmental factors, mainly exposure to antigens and the like, but environmental factors are believed to be a major cause of the recent increase in the prevalence of allergic diseases. While research on allergic diseases has gradually advanced our understanding of the onset mechanisms, aggravating factors and the like, there is still no complete prevention or curative treatment for allergic diseases, and the mainstay of treatment is long-term symptomatic treatment by securing the living environment such as avoiding antigens, and by drug therapy with histamine H1 antagonists, Th2 cytokine inhibitors, and the like. However, while for allergies to foreign substances such as pollen, it is relatively easy to avoid contact with the corresponding allergen, for food allergies, the corresponding allergens are often very importantly and widely used food, and avoiding contact with them is thus very difficult. In addition, long-term drug use is associated with side effects such as drowsiness, dry mouth, and gastrointestinal problems.

Therefore, from the viewpoint of safety, food and plant components with anti-allergic properties are being explored. One such component is linseed oil, the ingestion of which has been reported to suppress food allergy symptoms (Non Patent Literature 1). Linseed oil is pressed and refined from flaxseeds and is known to be rich in α-linolenic acid (ALA), an ω-3 highly unsaturated fatty acid.

On the other hand, fats or oils containing high concentrations of diacylglycerols have been reported to have physiological effects such as suppressing postprandial increases in blood triglycerides (neutral fat) and having little accumulation in the body. For example, Patent Literature 1 reports that fat or oil compositions containing 60 to 100% by weight of diglycerides, in which 15 to 90% by weight of the constituent fatty acids are ω-3 unsaturated fatty acids having less than 20 carbon atoms and the weight ratio of cis-ω-3 unsaturated fatty acids/(cis-ω-6 unsaturated fatty acids + saturated fatty acids + trans-unsaturated fatty acids) is 1 to 6, have excellent visceral fat-burning properties, body fat-burning properties, and the like.

However, it is not known that diacylglycerol-containing fats or oils, which contain high amounts of α-linolenic acid as a constituent fatty acid, have anti-allergic properties.

(Patent Literature 1) JP-A-2002-138296

(Non Patent Literature 1) Kunisawa et al., Sci Rep. 2015, 5, 9750

### Summary of the Invention

The present invention relates to the following 1) to 18) :
1) An anti-allergic agent comprising, as an active ingredient, a fat or oil in which a content of α-linolenic acid in fatty acids constituting the fat or oil is 40 mass% or more, and a content of diacylglycerol is 25 mass% or more.
2) An agent for suppressing IgE production comprising, as an active ingredient, a fat or oil in which a content of α-linolenic acid in fatty acids constituting the fat or oil is 40 mass% or more, and a content of diacylglycerol is 25 mass% or more.
3) An agent for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.
4) An anti-allergic food comprising, as an active ingredient, a fat or oil in which a content of α-linolenic acid in fatty acids constituting the fat or oil is 40 mass% or more, and a content of diacylglycerol is 25 mass% or more.
5) A food for suppressing IgE production comprising, as an active ingredient, a fat or oil in which a content of α-linolenic acid in fatty acids constituting the fat or oil is 40 mass% or more, and a content of diacylglycerol is 25 mass% or more.
6) A food for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.
7) Use of a fat or oil for producing an anti-allergic agent, in which a content of α-linolenic acid in fatty acids constituting the fat or oil is 40 mass% or more, and a content of diacylglycerol is 25 mass% or more.
8) A fat or oil for use against allergies, in which a content of α-linolenic acid in fatty acids constituting the fat or oil is 40 mass% or more, and a content of diacylglycerol is 25 mass% or more.
9) Non-therapeutic use of a fat or oil against allergies, in which a content of α-linolenic acid in fatty acids constituting the fat or oil is 40 mass% or more, and a content of diacylglycerol is 25 mass% or more.
10) Use of a fat or oil for producing an agent for suppressing IgE production, in which a content of α-linolenic acid in fatty acids constituting the fat or oil is 40 mass% or more, and a content of diacylglycerol is 25 mass% or more.
11) A fat or oil for use in suppressing IgE production in which a content of α-linolenic acid in fatty acids constituting the fat or oil is 40 mass% or more, and a content of diacylglycerol is 25 mass% or more.
12) Non-therapeutic use of a fat or oil for suppressing IgE production, in which a content of α-linolenic acid in fatty acids constituting the fat or oil is 40 mass% or more, and a content of diacylglycerol is 25 mass% or more.
13) Use of a fat or oil for producing an agent for suppressing Th2 cell response induced by allergen exposure, in which a content of α-linolenic acid in fatty acids constituting the fat or oil is 40 mass% or more, and a content of diacylglycerol is 25 mass% or more.
14) A fat or oil for use in suppressing Th2 cell response induced by allergen exposure, in which a content of α-linolenic acid in fatty acids constituting the fat or oil is 40 mass% or more, and a content of diacylglycerol is 25 mass% or more.
15) Non-therapeutic use of a fat or oil for suppressing Th2 cell response induced by allergen exposure, in which a content of α-linolenic acid in fatty acids constituting the fat or oil is 40 mass% or more, and a content of diacylglycerol is 25 mass% or more.
16) A method for preventing, treating, or improving allergies, comprising administering or applying, to a subject, a fat or oil, in which a content of α-linolenic acid in fatty acids constituting the fat or oil is 40 mass% or more, and a content of diacylglycerol is 25 mass% or more.
17) A method for suppressing IgE production, comprising administering or applying, to a subject, a fat or oil, in which a content of α-linolenic acid in fatty acids constituting the fat or oil is 40 mass% or more, and a content of diacylglycerol is 25 mass% or more.
18) A method for suppressing Th2 cell response induced by allergen exposure, comprising administering or applying, to a subject, a fat or oil, in which a content of α-linolenic acid in fatty acids constituting the fat or oil is 40 mass% or more, and a content of diacylglycerol is 25 mass% or more.

### Brief Description of Drawings

[Figure 1] Graph showing OVA-specific IgE levels in plasma.
[Figure 2] Graph showing IL-4 mRNA expression levels in inguinal lymph node cells.

### Detailed Description of the Invention

The present invention relates to providing an anti-allergic agent.

The present inventors used a food allergy model by transdermal sensitization to evaluate the effect of diacylglycerol-containing fats or oils with a high α-linolenic acid content in suppressing allergies. As a result, they found that the amount of allergen-specific IgE production in plasma was significantly reduced and IL-4 expression in inguinal lymph nodes was suppressed in mice raised on feed supplemented with diacylglycerol-containing fats or oils with a high α-linolenic acid content before food allergen sensitization, indicating that the fats or oils had anti-allergic properties.

The anti-allergic agent of the present invention suppresses IgE production and IL-4 expression, and thus has the potential of suppressing the onset of type I allergies or of alleviating allergic symptoms, in particular.

In the present invention, "fat or oil" refers to ester compounds of fatty acids and glycerol, and includes one or more of monoacylglycerol, diacylglycerol and triacylglycerol. The type of fat or oil is not particularly limited as long as it can be used as an edible fat or oil.

In the fat or oil used in the present invention, the content of α-linolenic acid in the fatty acids constituting the fat or oil is 40 mass% or more, and the content of diacylglycerol is 25 mass% or more. Hereinafter, the "fat or oil in which the content of **α-**linolenic acid in the fatty acids constituting the fat or oil is 40 mass% or more, and the content of diacylglycerol is 25 mass% or more" may be simply referred to as the "fat or oil of the present invention" in the present specification.

The content of α-linolenic acid in the fatty acids constituting the fat or oil of the present invention is 40 mass% or more, and from the viewpoint of physiological efficacy, it is preferably 45 mass% or more, more preferably 50 mass% or more, further more preferably 52 mass% or more, and from the viewpoint of oxidative stability, preferably 80 mass% or less, more preferably 70 mass% or less, further more preferably 60 mass% or less.

The content of α-linolenic acid in the fatty acids constituting the fat or oil is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less. The amount of fatty acid in the present specification is indicated in terms of free fatty acids.

In the present invention, the constituent fatty acids other than α-linolenic acid constituting the fat or oil are not particularly limited and may be either saturated fatty acids or unsaturated fatty acids.

From the viewpoint of flavor and industrial productivity of the fat or oil, the content of unsaturated fatty acids in the fatty acids constituting the fat or oil is preferably 60 mass% or more and 100 mass% or less, more preferably 70 mass% or more and 100 mass% or less, further more preferably 75 mass% or more and 99 mass% or less, further more preferably 80 mass% or more and 98 mass% or less. The number of carbon atoms in the unsaturated fatty acid is preferably from 14 to 24, more preferably from 16 to 22 from the viewpoint of physiological efficacy.

The content of linoleic acid (C18:2) in the fatty acids constituting the fat or oil is preferably 5 mass% or more, more preferably 10 mass% or more from the viewpoint of industrial productivity, and preferably 40 mass% or less, more preferably 30 mass% or less, further more preferably 20 mass% or less from the viewpoint of physiological efficacy.

The content of linoleic acid (C18:2) in the fatty acids constituting the fat or oil is preferably 5 mass% or more and 40 mass% or less, more preferably 10 mass% or more and 30 mass% or less, further more preferably 10 mass% or more and 20 mass% or less.

The content of oleic acid (C18:1) in the fatty acids constituting the fat or oil is preferably 10 mass% or more from the viewpoint of industrial productivity, and preferably 50 mass% or less, more preferably 40 mass% or less, further more preferably 30 mass% or less from the viewpoint of physiological efficacy.

The content of oleic acid (C18:1) in the fatty acids constituting the fat or oil is preferably 10 mass% or more and 50 mass% or less, more preferably 10 mass% or more and 40 mass% or less, further more preferably 10 mass% or more and 30 mass% or less.

The total content of saturated fatty acids in the fatty acids constituting the fat or oil is preferably 6.0 mass% or less, more preferably 5.5 mass% or less, further more preferably 5.0 mass% or less from the viewpoint of appearance, physiological efficacy, and industrial productivity of the fat or oil. From the viewpoint of industrial productivity, it is preferably 0.5 mass% or more.

The total content of saturated fatty acids in the fatty acids constituting the fat or oil is preferably 0.5 mass% or more and 6.0 mass% or less, further more preferably 0.5 mass% or more and 5.5 mass% or less, further more preferably 0.5 mass% or more and 5.0 mass% or less.

The type of saturated fatty acid is not particularly limited, but saturated fatty acids having from 14 to 24 carbon atoms are preferred, saturated fatty acids having from 16 to 22 carbon atoms are more preferred, and saturated fatty acids having 16, 18, and 20 carbon atoms are further more preferred.

The content of diacylglycerol in the fat or oil of the present invention is 25 mass% or more, and from the viewpoint of effective exhibition of the effect and physiological efficacy, it is preferably 30 mass% or more, more preferably 50 mass% or more, further more preferably 55 mass% or more, further more preferably 60 mass% or more, further more preferably 65 mass% or more, further more preferably 70 mass% or more, further more preferably 80 mass% or more, further more preferably 83 mass% or more, further more preferably 84 mass% or more, and preferably 96 mass% or less, more preferably 95 mass% or less, further more preferably 94 mass% or less.

The content of diacylglycerol in the fat or oil is preferably 25 mass% or more and 96 mass% or less, more preferably 30 mass% or more and 96 mass% or less, further more preferably 50 mass% or more and 96 mass% or less, further more preferably 50 mass% or more and 95 mass% or less, further more preferably 55 mass% or more and 95 mass% or less, further more preferably 60 mass% or more and 95 mass% or less, further more preferably 65 mass% or more and 95 mass% or less, further more preferably 65 mass% or more and 94 mass% or less, further more preferably 70 mass% or more and 94 mass% or less, further more preferably 80 mass% or more and 94 mass% or less, further more preferably 83 mass% or more and 94 mass% or less, further more preferably 84 mass% or more and 94 mass% or less.

In the present invention, the fatty acids constituting diacylglycerol may be either saturated fatty acids or unsaturated fatty acids, and the content of α-linolenic acid in the fatty acids constituting diacylglycerol is preferably 40 mass% or more from the viewpoint of effectively exhibiting the effect.

From the same viewpoint, the content of α-linolenic acid in the fatty acids constituting diacylglycerol is preferably 45 mass% or more, more preferably 50 mass% or more, further more preferably 52 mass% or more, and from the viewpoint of oxidative stability, preferably 80 mass% or less, more preferably 70 mass% or less, further more preferably 60 mass% or less.

The content of α-linolenic acid in the fatty acids constituting diacylglycerol is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.

The fat or oil of the present invention may contain triacylglycerol, the content of which is, from the viewpoint of industrial productivity of the fat or oil, preferably 1 mass% or more, more preferably 2 mass% or more, further more preferably 5 mass% or more, and preferably 75 mass% or less, more preferably 72 mass% or less, more preferably 50 mass% or less, more preferably 25 mass% or less.

The content of triacylglycerol in the fat or oil is preferably 1 mass% or more and 75 mass% or less, more preferably 2 mass% or more and 75 mass% or less, further more preferably 2 mass% or more and 72 mass% or less, further more preferably 5 mass% or more and 72 mass% or less, further more preferably 5 mass% or more and 50 mass% or less, further more preferably 5 mass% or more and 25 mass% or less.

From the viewpoint of flavor and industrial productivity of the fat or oil, the content of monoacylglycerol in the fat or oil is preferably 3 mass% or less, more preferably 2 mass% or less, further more preferably 1.5 mass% or less, and preferably more than 0 mass%. The content of monoacylglycerol in the fat or oil may be 0 mass%.

The fatty acid composition of triacylglycerol, diacylglycerol and monoacylglycerol is preferably the same from the viewpoint of industrial productivity of the fat or oil.

The fat or oil of the present invention may contain free fatty acids or salts thereof as impurities. The content of free fatty acids or salts thereof in the fat or oil is, from the viewpoint of flavor, preferably 3 mass% or less, more preferably 2 mass% or less, further more preferably 1 mass% or less, and preferably more than 0 mass%. The content of free fatty acids or salts thereof in the fat or oil may be 0 mass%.

The fat or oil of the present invention can be obtained by an esterification reaction between fatty acids derived from fats or oils and glycerol, a transesterification reaction between fats or oils and glycerol (glycerolysis), or the like in accordance with conventional methods. Conventional edible fats or oils may be mixed if necessary.

Esterification and glycerolysis reactions are roughly classified into chemical methods using chemical catalysts such as alkali metals or their alloys, oxides or hydroxides of alkali metals or alkaline earth metals, and alkoxides of alkali metals or alkaline earth metals, and enzymatic methods using enzymes such as lipases.

Of these, an esterification reaction between fatty acids obtained by hydrolyzing the fats or oils as described below (fractionated fatty acids) and glycerol is preferred from the viewpoint of controlling fatty acid composition.

In the present invention, the origin of the fats or oils is not particularly limited as long as they can be used as an edible fat or oil, and may be either from plants or animals. Example thereof include plant-derived fats or oils such as soybean oil, rapeseed oil, safflower oil, rice oil, corn oil, sunflower oil, cottonseed oil, olive oil, sesame oil, peanut oil, coix lacryma-jobi seed oil, wheat germ oil, perilla oil (shiso oil), linseed oil, perilla oil (egoma oil), chia seed oil, sacha inchi oil, walnut oil, kiwi seed oil, salvia seed oil, grape seed oil, macadamia nut oil, hazelnut oil, pumpkin seed oil, camellia oil, tea seed oil, borage oil, palm oil, palm olein, palm stearin, coconut oil, palm kernel oil, cacao butter, sal fat, shea butter, and algal oil; animal fats or oils such as fish oil, seal oil, lard, beef tallow, and butterfat; and transesterified oils thereof, hydrogenated oils thereof, fractionated oils thereof and the like.

These oils may be used alone, or in combination as appropriate. Of these, plant-derived fats or oils are preferably used from the viewpoint of usability, liquid fats or oils with excellent low-temperature resistance are further more preferably used, and one or more fats or oils selected from the group consisting of perilla oil (shiso oil), linseed oil, and perilla oil (egoma oil) are further more preferably used as they are rich in α-linolenic acid. Liquid fats or oils refer to fats or oils which are liquid at 20°C when subjected to a cooling test in accordance with the Standard Methods for the Analysis of Fats, Oils, and Related Materials 2.3.8-27. The edible fats or oils are preferably purified fats or oils which have undergone a purification step.

The fatty acids derived from the fats or oils can be obtained by hydrolyzing the fats or oils. Examples of methods for hydrolyzing the fats or oils include high temperature-pressure cleavage and enzymatic cleavage. High temperature-pressure cleavage is a method of adding water to the fat or oil and reacting them under high temperature and pressure conditions to obtain the fatty acids and glycerol. Enzymatic cleavage is a method of adding water to the fat or oil and reacting them under low temperature conditions using a fat or oil hydrolase to obtain the fatty acids and glycerol.

The hydrolysis reaction can be performed in accordance with a conventional method.

After hydrolysis of the fat or oil, the hydrolysates are preferably fractionated to remove any solids. Examples of fractionation methods include solvent fractionation, natural fractionation (dry fractionation), and wet fractionation.

Examples of the means for removing precipitated solids include static separation, filtration, centrifugation, and separation by mixing the fatty acids with an aqueous solution of a wetting agent.

The esterification reaction between the fatty acids derived from the fat or oil and glycerol is preferably performed under mild conditions by an enzymatic method, as it is superior from the viewpoint of flavor and the like.

The amount of enzyme used can be determined as appropriate considering the activity of the enzyme, but from the viewpoint of improving the reaction rate, it is preferably from 1 to 30 mass%, more preferably from 2 to 20 mass% of the total mass of raw materials for the esterification reaction when an immobilized enzyme is used.

The reaction temperature of the esterification reaction is preferably from 0 to 100°C, more preferably from 20 to 80°C, and further more preferably from 30 to 60°C, from the viewpoint of improving the reaction rate and suppressing enzyme deactivation. The reaction time is preferably within 15 hours, more preferably from 1 to 12 hours, and further more preferably from 2 to 10 hours, from the viewpoint of industrial productivity.

Examples of the means for bringing the fatty acids into contact with glycerol include immersion, agitation, and passing liquids through a column packed with immobilized lipase using a pump or the like.

After the esterification reaction, a purification step usually used for fats or oils may be performed. Specific examples include steps of distillation, acid treatment, washing with water, decolorization, and deodorization.

As shown in the Examples described below, the fat or oil of the present invention suppresses IgE production and also suppresses IL-4 expression in the inguinal lymph nodes. In general, allergens which have entered the body are taken up by antigen-presenting cells such as dendritic cells, and induce type 2 helper T cells (Th2 cells) through antigen presentation. Cytokines such as IL-4 produced by Th2 cells then activate B cells to produce IgE (Kucuksezer UC, Ozdemir C, Akdis M, Akdis CA. Mechanisms of immune tolerance to allergens in children. Korean J Pediatr. 2013 Dec;56(12) :505-13) . The above suggests that the fat or oil of the present invention exerts its anti-allergic effects via its effect of suppressing IgE production, particularly its effect of suppressing allergen-specific IgE production, based on the suppression of IL-4 production from Th2 cells.

Therefore, the administration or ingestion of the fat or oil of the present invention to animals, including humans, can suppress allergic reactions, particularly type I allergic reactions in the animals, including humans, and can suppress the onset of allergies or alleviate allergic symptoms.

Therefore, the fat or oil of the present invention can be an anti-allergic agent, an agent for suppressing IgE production, particularly an agent for suppressing allergen-specific IgE production, and an agent for suppressing Th2 cell response induced by allergen exposure (hereinafter also referred to as "anti-allergic agent and the like"), which can be used against allergies, for suppressing IgE production, particularly for suppressing allergen-specific IgE production, and for suppressing Th2 cell response induced by allergen exposure, and can be used to produce an anti-allergic agent and the like.

Here, "use" can be in humans or non-human animals, and may be therapeutic or non-therapeutic. "Non-therapeutic" is a concept which does not include medical practice, i.e., does not include methods of operating on, treating, or diagnosing a human being, and more specifically, does not include methods by which a physician, a healthcare professional or person directed by a physician performs surgery, treatment, or diagnosis on a human being.

In the present specification, examples of the targeted allergies include hay fever, allergic rhinitis, bronchial asthma, urticaria, allergic conjunctivitis, food allergies, anaphylactic shock, and drug allergies. It is particularly suitable for food allergies.

Examples of foods containing allergens include eggs, milk, meat (beef, chicken, pork, and the like), grains (soybeans, rice, wheat, buckwheat, sesame, peanuts, and the like), yams, seafood (abalone, squid, salmon roe, shrimp, crab, salmon, mackerel, and the like), and fruits (orange, kiwi fruit, peach, apple, fig, and the like). Examples of allergens include egg white albumin (ovalbumin) and ovomucoid in eggs, casein and β-lactoglobulin in milk, gliadin and α-amylase inhibitors in wheat, and 2S albumins in peanuts.

Examples of other substances containing allergens include molds (Alternaria, Penicillium, Candida, Cladosporium, Aspergillus, and the like), mites, and pollens (ragweed, Humulus japonicus, cedar, hinoki cypress, Japanese white birch, Japanese red pine, Japanese silver grass, southern cattail, and the like). Examples of the allergens include Alt a 1 in Alternaria mold, Asp f 1 in Aspergillus mold, Der f 1 and Der f 2 in mites, Amb a 1 in ragweed pollen, Cry j 1 and Cry j 2 in cedar pollen, and Bet v 1 in Japanese white birch pollen.

The anti-allergic agent and the like of the present invention can itself be a medicament, quasi-drug, cosmetic, food or feed against allergies, for suppressing IgE production, and for suppressing Th2 cell response induced by allergen exposure, or a material or preparation to be blended with the medicament, quasi-drug, cosmetic, food or feed.

The medicament (including quasi-drug, the same applies hereinafter) contains the fat or oil of the present invention as an active ingredient against allergies, for suppressing IgE production, and for suppressing Th2 cell response induced by allergen exposure. Furthermore, the medicament may contain a pharmaceutically acceptable carrier or other active ingredients, pharmacological ingredients, and the like, as needed, as long as the function of the active ingredient is not lost.

The medicament can be administered in any form of administration. Examples of forms of administration include oral solid preparations such as tablets (including chewable tablets), capsules, granules, powders, and lozenges; oral liquid preparations such as internal liquid medicines and syrups; and parenteral preparations such as injections, suppositories, inhalants, transdermal drugs, and topical products. The preferred form of administration is oral administration. The size of the form can be adjusted as desired according to the intended use.

These preparations of various dosage forms can be prepared in accordance with conventional methods by appropriately combining them with pharmaceutically acceptable carriers, such as excipients, binders, bulking agents, disintegrants, surfactants, lubricants, dispersants, buffers, osmotic pressure regulators, pH adjusters, emulsifiers, preservatives, stabilizers, preserving agents, thickeners, fluidity promoters, flavoring agents, foaming agents, flavors, coating agents, and diluents, or other pharmaceutical ingredients, as necessary.

The cosmetic contains the fat or oil of the present invention as an active ingredient against allergies, for suppressing IgE production, and for suppressing Th2 cell response induced by allergen exposure. Furthermore, the cosmetic may contain a cosmetically acceptable carrier or other active ingredients, pharmaceutical ingredients, cosmetic ingredients, and the like, as needed, as long as the function of the active ingredient is not lost.

Preferred examples of cosmetics containing the fat or oil of the present invention include cosmetics for the face and body (e.g., lotions, gels, creams, and facial masks), make-up cosmetics, and face or body cleansers.

Each of these cosmetic preparations can be produced in accordance with conventional methods. Examples of cosmetically acceptable carriers include various oils, surfactants, gelling agents, buffers, preservatives, antioxidants, solvents, dispersants, chelating agents, thickeners, ultraviolet absorbers, emulsion stabilizers, pH adjusters, pigments, and flavors.

Examples of other active, pharmaceutical, and cosmetic ingredients include plant extracts, germicides, moisturizing agents, anti-inflammatory agents, antibacterial agents, keratolytic agents, cooling agents, anti-seborrheic agents, cleansing agents, and makeup ingredients.

The food contains the fat or oil of the present invention as an active ingredient against allergies, for suppressing IgE production, and for suppressing Th2 cell response induced by allergen exposure.

The food includes those with an anti-allergy claim and for which labeling to that effect has been approved or notified as necessary (food for specified health uses and food with functional claims). Examples of labels include "relieves discomfort in the eyes and nose." Foods for which functional claims are approved or notified can be distinguished from common foods.

The form of the food can be solid, semi-solid or liquid (e.g., beverage). Examples thereof include various food compositions (breads, cakes, noodles, confectioneries, frozen foods, ice cream, candy, furikake, soups, dairy products, shakes, beverages, seasonings, and the like), as well as nutritional supplement compositions in the same forms as the oral preparations described above (solid preparations such as granules, powders, tablets, capsules, microcapsules, and lozenges).

Foods in various forms can be prepared in accordance with conventional methods by appropriately combining the fat or oil of the present invention with food material, or other active ingredients, or food-acceptable additives (e.g., solvents, softeners, oils, emulsifiers, preservatives, acidulants, sweeteners, bittering agents, pH adjusters, stabilizers, coloring agents, ultraviolet absorbers, antioxidants, moisturizing agents, thickeners, fixing agents, dispersants, fluidity promoters, wetting agents, flavors, seasonings, and flavor modifiers) as desired.

The feed contains the fat or oil of the present invention as an active ingredient against allergies, for suppressing IgE production, and for suppressing Th2 cell response induced by allergen exposure.

The feed is preferably in the form of pellets, flakes, a mash or liquid, and examples thereof include livestock feed used for cattle, pigs, chickens, sheep, horses, and the like, feed for small animals used for rabbits, rats, mice, and the like, and pet food used for dogs, cats, small birds, and the like.

The feed can be prepared in accordance with conventional methods by appropriately combining the fat or oil of the present invention with other feed materials, such as meat, protein, grains, bran, sake lees, sugars, vegetables, vitamins, minerals, gelling agents, shape-retaining agents, pH adjusters, seasonings, preservatives, and nutritional enhancers.

The content of the fat or oil of the present invention in the preparation cannot be generalized as it varies depending on the form of the preparation, but for example, it is preferably 0.5 mass% or more, and preferably 99.8 mass% or less based on the total amount of the preparation.

Any dose or amount used of the fat or oil of the present invention can be used as long as it achieves the effect of the present invention. The dose or amount used can vary according to the subject' species, weight, sex, age, condition, or other factors, but in the case of oral administration (ingestion), it is, for example, from 0.3 to 15 g of the fat or oil of the present invention per adult (60 kg) per dose.

The above preparation can be administered or used according to any dosage regimen, and can be administered or used repeatedly and continuously for 1 day or more, preferably for 7 days or more, more preferably for 14 days or more, even more preferably for 30 days or more, in a single or a plurality of doses per day.

The anti-allergic agent and the like of the present invention is preferably administered or used in the form of a fat or oil composition.

If the anti-allergic agent and the like is in the form of a fat or oil composition, the content of the fat or oil of the present invention in the fat or oil composition is preferably 90 mass% or more, more preferably 95 mass% or more, and preferably 100 mass% or less, more preferably 99.9 mass% or less, from the viewpoint of usability.

The fat or oil composition preferably contains an antioxidant from the viewpoint of flavor, oxidation stability, suppression of coloration, and the like. The content of antioxidant in the fat or oil composition is preferably 0.005 mass% or more and 1.0 mass% or less, more preferably 0.04 mass% or more and 0.75 mass% or less, further more preferably 0.08 mass% or more and 0.5 mass% or less, from the viewpoint of flavor, oxidation stability, suppression of coloration, and the like.

The antioxidant is not particularly limited as long as it is used in foods, but is preferably at least one selected from the group consisting of natural antioxidants, lecithin, tocopherol, ascorbyl palmitate, ascorbyl stearate, dibutylhydroxytoluene (BHT) and butylhydroxyanisole (BHA).

Examples of the subjects to whom the anti-allergic agent and the like of the present invention is administered or used include humans who wish to suppress the onset of allergies and humans who wish to alleviate allergic symptoms. Preferred examples include humans who wish to suppress the onset of food allergies and humans who wish to alleviate symptoms of food allergies. Examples of non-human animals include non-human mammals such as anthropoid apes and other primates.

With respect to the embodiments described above, the present invention further discloses the following aspects.

<1> An anti-allergic agent comprising, as an active ingredient, a fat or oil in which a content of α-linolenic acid in fatty acids constituting the fat or oil is 40 mass% or more, and a content of diacylglycerol is 25 mass% or more.
<2> An agent for suppressing IgE production comprising, as an active ingredient, a fat or oil in which a content of α-linolenic acid in fatty acids constituting the fat or oil is 40 mass% or more, and a content of diacylglycerol is 25 mass% or more.
<3> The agent for suppressing IgE production according to <2>, wherein the agent is for suppressing allergen-specific IgE production.
<4> An agent for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.
<5> An anti-allergic food comprising, as an active ingredient, a fat or oil in which a content of α-linolenic acid in fatty acids constituting the fat or oil is 40 mass% or more, and a content of diacylglycerol is 25 mass% or more.
<6> A food for suppressing IgE production comprising, as an active ingredient, a fat or oil in which a content of α-linolenic acid in fatty acids constituting the fat or oil is 40 mass% or more, and a content of diacylglycerol is 25 mass% or more.
<7> The food for suppressing IgE production according to <6>, wherein the food is for suppressing allergen-specific IgE production.
<8> A food for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.
<9> The agent or food according to any one of <1> to <8>, wherein the content of α-linolenic acid in the fatty acids constituting the fat or oil is preferably 45 mass% or more, more preferably 50 mass% or more, further more preferably 52 mass% or more, preferably 80 mass% or less, more preferably 70 mass% or less, further more preferably 60 mass% or less, and is 40 mass% or more and 80 mass% or less, preferably 45 mass% or more and 80 mass% or less, more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.
<10> The agent or food according to any one of <1> to <9>, wherein a content of linoleic acid (C18:2) in the fatty acids constituting the fat or oil is preferably 5 mass% or more, more preferably 10 mass% or more, preferably 40 mass% or less, more preferably 30 mass% or less, further more preferably 20 mass% or less, and preferably 5 mass% or more and 40 mass% or less, more preferably 10 mass% or more and 30 mass% or less, further more preferably 10 mass% or more and 20 mass% or less.
<11> The agent or food according to any one of <1> to <10>, wherein a content of oleic acid (C18:1) in the fatty acids constituting the fat or oil is preferably 10 mass% or more, preferably 50 mass% or less, more preferably 40 mass% or less, further more preferably 30 mass% or less, and preferably 10 mass% or more and 50 mass% or less, more preferably 10 mass% or more and 40 mass% or less, further more preferably 10 mass% or more and 30 mass% or less.
<12> The agent or food according to any one of <1> to <11>, wherein a content of diacylglycerol in the fat or oil is preferably 30 mass% or more, more preferably 50 mass% or more, further more preferably 55 mass% or more, further more preferably 60 mass% or more, further more preferably 65 mass% or more, further more preferably 70 mass% or more, further more preferably 80 mass% or more, further more preferably 83 mass% or more, further more preferably 84 mass% or more, preferably 96 mass% or less, more preferably 95 mass% or less, further more preferably 94 mass% or less, and preferably 25 mass% or more and 96 mass% or less, more preferably 30 mass% or more and 96 mass% or less, further more preferably 50 mass% or more and 96 mass% or less, further more preferably 50 mass% or more and 95 mass% or less, further more preferably 55 mass% or more and 95 mass% or less, further more preferably 60 mass% or more and 95 mass% or less, further more preferably 65 mass% or more and 95 mass% or less, further more preferably 65 mass% or more and 94 mass% or less, further more preferably 70 mass% or more and 94 mass% or less, further more preferably 80 mass% or more and 94 mass% or less, further more preferably 83 mass% or more and 94 mass% or less, further more preferably 84 mass% or more and 94 mass% or less.
<13> The agent or food according to any one of <1> to <12>, wherein a content of α-linolenic acid in fatty acids constituting the diacylglycerol is preferably 40 mass% or more, more preferably 45 mass% or more, further more preferably 50 mass% or more, further more preferably 52 mass% or more, preferably 80 mass% or less, more preferably 70 mass% or less, further more preferably 60 mass% or less, and preferably 40 mass% or more and 80 mass% or less, more preferably 45 mass% or more and 80 mass% or less, further more preferably 50 mass% or more and 80 mass% or less, more preferably 50 mass% or more and 70 mass% or less, further more preferably 52 mass% or more and 70 mass% or less, further more preferably 52 mass% or more and 60 mass% or less.
<14> The agent or food according to any one of <1>, <5>, and <9> to <13>, wherein the allergy is preferably a type I allergy, more preferably hay fever, allergic rhinitis, bronchial asthma, urticaria, allergic conjunctivitis, food allergy, anaphylactic shock, or drug allergy, further more preferably food allergy.
<15> The agent or food according to any one of <3>, <4>, and <7> to <13>, wherein the allergen is preferably egg, milk, meat, grain, yam, seafood, fruit, mold, mite, or pollen.
<16> Use of a fat or oil for producing an anti-allergic agent, in which a content of α-linolenic acid in fatty acids constituting the fat or oil is 40 mass% or more, and a content of diacylglycerol is 25 mass% or more.
<17> A fat or oil for use against allergies, in which a content of α-linolenic acid in fatty acids constituting the fat or oil is 40 mass% or more, and a content of diacylglycerol is 25 mass% or more.
<18> Non-therapeutic use of a fat or oil against allergies, in which a content of α-linolenic acid in fatty acids constituting the fat or oil is 40 mass% or more, and a content of diacylglycerol is 25 mass% or more.
<19> The fat or oil or use according to any one of <16> to <18>, wherein the allergy is preferably a type I allergy, more preferably hay fever, allergic rhinitis, bronchial asthma, urticaria, allergic conjunctivitis, food allergy, anaphylactic shock, or drug allergy, further more preferably food allergy.
<20> Use of a fat or oil for producing an agent for suppressing IgE production, in which a content of α-linolenic acid in fatty acids constituting the fat or oil is 40 mass% or more, and a content of diacylglycerol is 25 mass% or more.
<21> The use of the fat or oil according to <20>, which is the production of an agent for suppressing allergen-specific IgE production.
<22> A fat or oil for use in suppressing IgE production, in which a content of α-linolenic acid in fatty acids constituting the fat or oil is 40 mass% or more, and a content of diacylglycerol is 25 mass% or more.
<23> The fat or oil according to <22>, for use in suppressing allergen-specific IgE production.
<24> Non-therapeutic use of a fat or oil for suppressing IgE production, in which a content of α-linolenic acid in fatty acids constituting the fat or oil is 40 mass% or more, and a content of diacylglycerol is 25 mass% or more.
<25> The non-therapeutic use of the fat or oil according to <24>, for suppressing allergen-specific IgE production.
<26> Use of a fat or oil for producing an agent for suppressing Th2 cell response induced by allergen exposure, in which a content of α-linolenic acid in fatty acids constituting the fat or oil is 40 mass% or more, and a content of diacylglycerol is 25 mass% or more.
<27> A fat or oil for use in suppressing Th2 cell response induced by allergen exposure, in which a content of α-linolenic acid in fatty acids constituting the fat or oil is 40 mass% or more, and a content of diacylglycerol is 25 mass% or more.
<28> Non-therapeutic use of a fat or oil for suppressing Th2 cell response induced by allergen exposure, in which a content of α-linolenic acid in fatty acids constituting the fat or oil is 40 mass% or more, and a content of diacylglycerol is 25 mass% or more.
<29> The fat or oil or use according to any one of <16> to <28>, wherein a content of α-linolenic acid in the fatty acids constituting diacylglycerol is 40 mass% or more.
<30> The fat or oil or use according to any one of <21>, <23>, and <25> to <29>, wherein the allergen is preferably egg, milk, meat, grain, yam, seafood, fruit, mold, mite, or pollen.
<31> A method for preventing, treating, or improving an allergy, wherein a fat or oil in which a content of α-linolenic acid in the fatty acids constituting the fat or oil is 40 mass% or more, and a content of diacylglycerol is 25 mass% or more, is administered or applied to a subject.
<32> The method according to <31>, wherein the allergy is preferably a type I allergy, more preferably hay fever, allergic rhinitis, bronchial asthma, urticaria, allergic conjunctivitis, food allergy, anaphylactic shock, or drug allergy, further more preferably food allergy.
<33> A method for suppressing IgE production, comprising administering or applying, to a subject, a fat or oil in which a content of α-linolenic acid in the fatty acids constituting the fat or oil is 40 mass% or more, and a content of diacylglycerol is 25 mass% or more.
<34> The method for suppressing IgE production according to <33>, wherein the method is for suppressing allergen-specific IgE production.
<35> A method for suppressing Th2 cell response induced by allergen exposure, comprising administering or applying, to a subject, a fat or oil in which a content of α-linolenic acid in fatty acids constituting the fat or oil is 40 mass% or more, and a content of diacylglycerol is 25 mass% or more, is administered or applied to a subject.
<36> The method according to any one of <31> to <35>, wherein a content of α-linolenic acid in the fatty acids constituting diacylglycerol is 40 mass% or more.
<37> The method according to any one of <34> to <36>, wherein the allergen is preferably egg, milk, meat, grain, yam, seafood, fruit, mold, mite, or pollen.

### Examples

### Example 1

### [Preparation of fat or oil and specialty feed]

The fatty acids obtained by hydrolyzing linseed oil (manufactured by Archer Daniels Midland) with an enzyme were fractionated by cooling and centrifugation to obtain fractionated fatty acids. 300 parts by mass of the obtained fractionated fatty acid was mixed with 47 parts by mass of glycerol, and an esterification reaction was performed using a commercially available immobilized 1,3-regioselective lipase (Novozymes) as a catalyst. After filtration of the immobilized enzyme, the reaction product was molecularly distilled, treated with acid, and washed with water to obtain a treated oil. The treated oil was deodorized, and then an antioxidant was added to prepare a fat or oil containing diacylglycerol (DAG) derived from linseed oil (lin-DAG).

The linseed oil-derived DAG fat or oil (lin-DAG) prepared in accordance with the above method, linseed oil (lin-TAG, manufactured by Summit oil mill), and as a control, soybean oil (soy-TAG, manufactured by The Nisshin OilliO Group, Ltd.) were prepared. The same concentration of the same antioxidant as lin-DAG was also added to lin-TAG and soy-TAG. Three types of specialty feed were prepared by adding 10 mass% of any of the above fats or oils to AIN-93G (Oriental Yeast Co., ltd.), which is used as mouse feed and from which the soybean oil (7 mass%) and cornstarch (3 mass%) that had been originally contained were removed.

The glyceride and fatty acid compositions of lin-DAG, lin-TAG and soy-TAG are shown in Table 1. The C18:3 unsaturated fatty acid in the fatty acid composition of Table 1 is α-linolenic acid.

**[Table 1]**

| Analysis items | | | lin-DAG | lin-TAG | soy-TAG |
|---|---|---|---|---|---|
| Glyceride composition | MAG | [%] | 1.4 | 0.0 | 0.1 |
| | DAG | [%] | 84.3 | 2.5 | 1.4 |
| | TAG | [%] | 13.9 | 97.5 | 98.5 |
| Fatty acid composition | C14:0 | [%] | 0.0 | 0.1 | 0.1 |
| | C16:0 | [%] | 2.7 | 5.6 | 10.6 |
| | C16:1 | [%] | 0.1 | 0.1 | 0.1 |
| | C18:0 | [%] | 1.6 | 4.8 | 3.9 |
| | C18:1 | [%] | 23.2 | 19.9 | 24.8 |
| | C18:2 | [%] | 16.0 | 15.8 | 51.7 |
| | C18:3 | [%] | 54.8 | 52.5 | 7.1 |
| | C20:0 | [%] | 0.1 | 0.2 | 0.4 |
| | C20:5 | [%] | 0 | 0 | 0 |
| | C22:0 | [%] | 0.2 | 0.2 | 0.5 |
| | C22:6 | [%] | 0 | 0 | 0 |
| | Others | [%] | 1.2 | 1.0 | 0.9 |

The methods for analyzing glyceride and fatty acid compositions are as follows.

### (i) Glyceride composition of fat or oil

To a glass sample bottle were added about 10 mg of fat or oil sample and 0.5 mL of trimethylsilylating agent ("Silylating Agent TH", manufactured by Kanto Chemical). The bottle was sealed tightly, and heated at 70°C for 15 minutes. To this was added 1.0 mL of water and 1.5 mL of hexane, and the mixture was shaken. After leaving it to stand, the upper layer was subjected to gas chromatography (GLC) for analysis.

### <GLC Analysis Conditions>

### (Conditions)

Apparatus: Agilent 6890 series (manufactured by Agilent Technologies)
Integrator: ChemStation B 02.01 SR2 (manufactured by Agilent Technologies)
Column: DB-1ht 10 m x 0.25 mm x 0.2 µm (manufactured by Agilent J&W)
Carrier gas: 1.0 mL He/min
Injector: Split (1:50), T = 340°C
Injection volume: 1 µL
Detector: FID, T = 350°C
Oven temperature: Raised from 80°C to 340°C at 10°C/minute and held for 15 minutes

### (ii) Constituent fatty acid composition of fat or oil

Fatty acid methyl esters were prepared in accordance with the "Method for Preparation of Fatty Acid Methyl Esters (2.4.1.-1996)" in "Standard Methods for the Analysis of Fats, Oils, and Related Materials" edited by the Japan Oil Chemists' Society, and the obtained fat or oil samples were measured in accordance with the American Oil Chemists' Society Official Method Ce 1f-96 (GLC method).

### <GLC Analysis Conditions>

Column: CP-SIL88 50 m x 0.25 mm x 0.2 µm (VARIAN)
Carrier gas: 1.0 mL He/min
Injector: Split (1:50), T = 300°C
Injection volume: 1 µL
Detector: FID, T = 300°C
Oven temperature: 150°C held for 5 min → 1°C/min temperature increase → 160°C held for 5 min → 2°C/min temperature increase → 200°C held for 10 min → 10°C/min temperature increase → 220°C held for 5 min

The fatty acid compositions of diacylglycerol in lin-DAG, lin-TAG and soy-TAG are shown in Table 2.

**[Table 2]**

| Analysis items | | | lin-DAG | lin-TAG | soy-TAG |
|---|---|---|---|---|---|
| Fatty acid composition | C14:0 | [%] | 0 | 0 | 0 |
| | C16:0 | [%] | 2.5 | 6.9 | 13.4 |
| | C16:1 | [%] | 0.1 | 0 | 0 |
| | C18:0 | [%] | 1.4 | 4.8 | 5.5 |
| | C18:1 | [%] | 23.0 | 25.1 | 19.5 |
| | C18:2 | [%] | 16.0 | 21.7 | 50.5 |
| | C18:3 | [%] | 56.3 | 38.1 | 5.1 |
| | C20:0 | [%] | 0.1 | 0.5 | 0.9 |
| | C20:5 | [%] | 0 | 0 | 0 |
| | C22:0 | [%] | 0.1 | 0.8 | 1.3 |
| | C22:6 | [%] | 0 | 0 | 0 |
| | Others | [%] | 0.6 | 2.2 | 3.7 |

The method for analyzing the fatty acid composition of diacylglycerol is as follows.

The DAG fraction was extracted from each fat or oil sample and derivatized, then subjected to gas chromatography (GLC method) for analysis under the following conditions. In this instance, the measurement method was based on the American Oil Chemists' Society (AOCS) Official Method Ce 1f-96. The C18:3 unsaturated fatty acid in the fatty acid composition of Table 2 is α-linolenic acid.

### <GLC Analysis Conditions>

Apparatus: Agilent 7890B (manufactured by Agilent Technologies)
Column: CP-Sil 88 for FAME 50 m x 0.25 mm x 0.2 µm (manufactured by Agilent Technologies)
Carrier gas: 1.0 mL He/min
Injector: Split (1:50), T = 300°C
Injection volume: 1 µL
Detector: FID, T = 300°C
Oven temperature: 150°C held for 5 min → 1°C/min temperature increase → 160°C held for 5 min → 2°C/min temperature increase → 200°C held for 10 min → 10°C/min temperature increase →220°C held for 5 min

### [Test Overview]

(1) 4 to 6-week-old BALB/c female mice were divided into a non-sensitized group (soy-TAG (-) group) and a transdermally sensitized group (soy-TAG (+) group, lin-TAG (+) group and lin-DAG (+) group) (N=8), and the mice of each group were fed the above specialty feed ad libitum. After 30 days, the backs of the mice in the transdermally sensitized group were shaved and coated with a 4 mass% sodium dodecyl sulfate (SDS) solution, and then 300 µg of egg white albumin (OVA) dissolved in 60 µL of phosphate buffer solution (PBS solution) was applied for transdermal sensitization. This transdermal sensitization was repeated three times a week for a total of two weeks. 21 days after the start of transdermal sensitization, 10 mg of OVA dissolved in 200 µL of PBS solution was orally administered to induce a food allergy. After collecting blood on the following day, the mice were euthanized and their inguinal lymph nodes collected. The mice in the non-sensitized group were orally administered PBS solution alone instead of OVA.

During the animal experiment, body weight was measured once a week and food intake once every 2 or 3 days, but no differences were observed among the groups.

### (2) Measurement of OVA-specific IgE concentration

Plasma was separated from the collected blood. OVA-specific IgE concentrations in plasma were measured using the LBIS OVA-IgE Mouse Kit (FUJIFILM Wako Shibayagi Corporation) in accordance with the accompanying protocol.

### (3) Quantification of mRNA expression levels of IL-4

The collected inguinal lymph nodes were impregnated with RLT buffer, crushed with a pencil mixer, and then subjected to total RNA extraction using an RNeasy Mini kit (Qiagen) in accordance with the accompanying protocol. cDNA was synthesized from the obtained total RNA using a SuperScript (registered trademark) VILO (registered trademark) cDNA Synthesis Kit (invitrogen). RT-qPCR was performed using the synthesized cDNA to quantify the mRNA expression levels of IL-4. In this instance, RPLP0 was used as an endogenous control.

### (4) Results

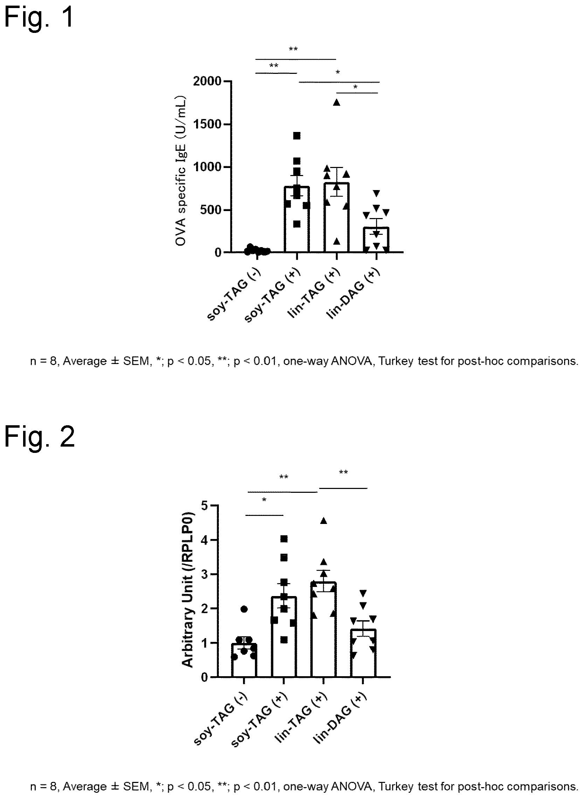

The results are shown in Figures 1 and 2.

As shown in Figure 1, compared to the non-sensitized soy-TAG (-) group, increased OVA-specific IgE production was observed in the transdermally sensitized soy-TAG (+) and lin-TAG (+) groups fed with the specialty feed containing soy-TAG and lin-TAG, while OVA-specific IgE production was significantly reduced in the lin-DAG (+) group fed with the specialty feed containing lin-DAG. As shown in Figure 2, compared to the non-sensitized soy-TAG (-) group, increased IL-4 mRNA expression levels in the inguinal lymph node cells were observed in the transdermally sensitized soy-TAG (+) and lin-TAG (+) groups, while IL-4 expression was suppressed in the lin-DAG group (+).

## Claims

1. An anti-allergic agent comprising, as an active ingredient, a fat or oil in which a content of α-linolenic acid in fatty acids constituting the fat or oil is 40 mass% or more, and a content of diacylglycerol is 25 mass% or more.

2. The anti-allergic agent according to claim 1, wherein an allergy is a type I allergy.

3. The anti-allergic agent according to claim 1 or 2, wherein the allergy is hay fever, allergic rhinitis, bronchial asthma, urticaria, allergic conjunctivitis, food allergy, anaphylactic shock, or drug allergy.

4. The anti-allergic agent according to any one of claims 1 to 3, wherein a content of α-linolenic acid in the fatty acids constituting diacylglycerol is 40 mass% or more.

5. An agent for suppressing IgE production comprising, as an active ingredient, a fat or oil in which a content of α-linolenic acid in fatty acids constituting the fat or oil is 40 mass% or more, and a content of diacylglycerol is 25 mass% or more.

6. The agent for suppressing IgE production according to claim 5, wherein the agent is for suppressing allergen-specific IgE production.

7. The agent for suppressing IgE production according to claim 5 or 6, wherein a content of α-linolenic acid in the fatty acids constituting diacylglycerol is 40 mass% or more.

8. An agent for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.

9. The agent for suppressing Th2 cell response induced by allergen exposure according to claim 8,
wherein a content of α-linolenic acid in the fatty acids constituting diacylglycerol is 40 mass% or more.

10. An anti-allergic food comprising, as an active ingredient, a fat or oil in which a content of α-linolenic acid in fatty acids constituting the fat or oil is 40 mass% or more, and a content of diacylglycerol is 25 mass% or more.

11. The anti-allergic food according to claim 10, wherein an allergy is a type I allergy.

12. The anti-allergic food according to claim 10 or 11, wherein the allergy is hay fever, allergic rhinitis, bronchial asthma, urticaria, allergic conjunctivitis, food allergy, anaphylactic shock, or drug allergy.

13. The anti-allergic food according to any one of claims 10 to 12, wherein a content of α-linolenic acid in the fatty acids constituting diacylglycerol is 40 mass% or more.

14. A food for suppressing IgE production comprising, as an active ingredient, a fat or oil in which a content of α-linolenic acid in fatty acids constituting the fat or oil is 40 mass% or more, and a content of diacylglycerol is 25 mass% or more.

15. The food for suppressing IgE production according to claim 14, wherein the food is for suppressing allergen-specific IgE production.

16. The food for suppressing IgE production according to claim 14 or 15, wherein a content of α-linolenic acid in the fatty acids constituting diacylglycerol is 40 mass% or more.

17. A food for suppressing Th2 cell response induced by allergen exposure comprising, as an active ingredient, a fat or oil in which a content of α-linolenic acid in fatty acids constituting the fat or oil is 40 mass% or more, and a content of diacylglycerol is 25 mass% or more.

18. The food for suppressing Th2 cell response induced by allergen exposure according to claim 17,
wherein a content of α-linolenic acid in the fatty acids constituting diacylglycerol is 40 mass% or more.

19. Use of a fat or oil for producing an anti-allergic agent in which a content of α-linolenic acid in fatty acids constituting the fat or oil is 40 mass% or more, and a content of diacylglycerol is 25 mass% or more.

20. The use according to claim 19, wherein an allergy is a type I allergy.

21. The use according to claim 19 or 20, wherein the allergy is hay fever, allergic rhinitis, bronchial asthma, urticaria, allergic conjunctivitis, food allergy, anaphylactic shock, or drug allergy.

22. The use according to any one of claims 19 to 21, wherein a content of α-linolenic acid in the fatty acids constituting diacylglycerol is 40 mass% or more.

23. Use of a fat or oil for producing an agent for suppressing IgE production in which a content of α-linolenic acid in fatty acids constituting the fat or oil is 40 mass% or more, and a content of diacylglycerol is 25 mass% or more.

24. The use according to claim 23, wherein the use is for producing an agent for suppressing allergen-specific IgE production.

25. The use according to claim 23 or 24,
wherein a content of α-linolenic acid in the fatty acids constituting diacylglycerol is 40 mass% or more.

26. Use of a fat or oil for producing an agent for suppressing Th2 cell response induced by allergen exposure in which a content of α-linolenic acid in fatty acids constituting the fat or oil is 40 mass% or more, and a content of diacylglycerol is 25 mass% or more.

27. The use according to claim 26, wherein a content of α-linolenic acid in the fatty acids constituting diacylglycerol is 40 mass% or more.

28. A fat or oil for use against an allergy in which a content of α-linolenic acid in fatty acids constituting the fat or oil is 40 mass% or more, and a content of diacylglycerol is 25 mass% or more.

29. The fat or oil according to claim 28,
wherein the allergy is a type I allergy.

30. The fat or oil according to claim 28 or 29, wherein the allergy is hay fever, allergic rhinitis, bronchial asthma, urticaria, allergic conjunctivitis, food allergy, anaphylactic shock, or drug allergy.

31. The fat or oi according to any one of claims 28 to 30, wherein a content of α-linolenic acid in the fatty acids constituting diacylglycerol is 40 mass% or more.

32. A fat or oil for use in suppressing IgE production in which a content of α-linolenic acid in fatty acids constituting the fat or oil is 40 mass% or more, and a content of diacylglycerol is 25 mass% or more.

33. The fat or oil according to claim 32, wherein the fat or oil is for use in suppressing allergen-specific IgE production.

34. The fat or oil according to claim 32 or 33, wherein a content of α-linolenic acid in the fatty acids constituting diacylglycerol is 40 mass% or more.

35. A fat or oil for use in suppressing Th2 cell response induced by allergen exposure in which a content of α-linolenic acid in fatty acids constituting the fat or oil is 40 mass% or more, and a content of diacylglycerol is 25 mass% or more.

36. The fat or oil according to claim 35, wherein a content of α-linolenic acid in the fatty acids constituting diacylglycerol is 40 mass% or more.

37. Non-therapeutic use of a fat or oil against an allergy in which a content of α-linolenic acid in fatty acids constituting the fat or oil is 40 mass% or more, and a content of diacylglycerol is 25 mass% or more.

38. The non-therapeutic use according to claim 37, wherein the allergy is a type I allergy.

39. The non-therapeutic use according to claim 37 or 38, wherein the allergy is hay fever, allergic rhinitis, bronchial asthma, urticaria, allergic conjunctivitis, food allergy, anaphylactic shock, or drug allergy.

40. The non-therapeutic use according to any one of claims 37 to 39, wherein a content of α-linolenic acid in the fatty acids constituting diacylglycerol is 40 mass% or more.

41. Non-therapeutic use of a fat or oil for suppressing IgE production in which a content of **α-**linolenic acid in fatty acids constituting the fat or oil is 40 mass% or more, and a content of diacylglycerol is 25 mass% or more.

42. The non-therapeutic use according to claim 41, wherein the non-therapeutic use is for suppressing allergen-specific IgE production.

43. The non-therapeutic use according to claim 41 or 42, wherein a content of α-linolenic acid in the fatty acids constituting diacylglycerol is 40 mass% or more.

44. Non-therapeutic use of a fat or oil for suppressing Th2 cell response induced by allergen exposure in which a content of α-linolenic acid in fatty acids constituting the fat or oil is 40 mass% or more, and a content of diacylglycerol is 25 mass% or more.

45. The non-therapeutic use according to claim 44, wherein a content of α-linolenic acid in the fatty acids constituting diacylglycerol is 40 mass% or more.

46. A method for preventing, treating, or improving an allergy, comprising administering or applying, to a subject, a fat or oil in which a content of α-linolenic acid in fatty acids constituting the fat or oil is 40 mass% or more, and a content of diacylglycerol is 25 mass% or more.

47. The method according to claim 46, wherein the allergy is a type I allergy.

48. The method according to claim 46 or 47, wherein the allergy is hay fever, allergic rhinitis, bronchial asthma, urticaria, allergic conjunctivitis, food allergy, anaphylactic shock, or drug allergy.

49. The method according to any one of claims 46 to 48, wherein a content of α-linolenic acid in the fatty acids constituting diacylglycerol is 40 mass% or more.

50. A method for suppressing IgE production, comprising administering or applying, to a subject, a fat or oil in which a content of α-linolenic acid in fatty acids constituting the fat or oil is 40 mass% or more, and a content of diacylglycerol is 25 mass% or more, is administered or applied to a subject.

51. The method according to claim 50, wherein the method is for suppressing allergen-specific IgE production.

52. The method according to claim 50 or 51, wherein a content of α-linolenic acid in the fatty acids constituting diacylglycerol is 40 mass% or more.

53. A method for suppressing Th2 cell response induced by allergen exposure, comprising administering or applying, to a subject, a fat or oil in which a content of α-linolenic acid in fatty acids constituting the fat or oil is 40 mass% or more, and a content of diacylglycerol is 25 mass% or more.

54. The method according to claim 53, wherein a content of α-linolenic acid in the fatty acids constituting diacylglycerol is 40 mass% or more.
